(19) Europäisches Patentamt
European Patent Office
Office européen des brevets

(11) **EP 3 430 002 B1**

(12) **FASCICULE DE BREVET EUROPEEN**

(45) Date de publication et mention de la délivrance du brevet:
**29.12.2021 Bulletin 2021/52**

(21) Numéro de dépôt: **17710561.6**

(22) Date de dépôt: **16.03.2017**

(51) Int Cl.:
***C07D 307/68*** *(2006.01)*

(86) Numéro de dépôt international:
**PCT/EP2017/056264**

(87) Numéro de publication internationale:
**WO 2017/158106 (21.09.2017 Gazette 2017/38)**

(54) **PROCEDE DE PREPARATION D'ACIDE FUROÏQUE**

FURANSÄUREHERSTELLUNGSVERFAHREN

FUROIC ACID PREPARATION METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.03.2016 FR 1652217**

(43) Date de publication de la demande:
**23.01.2019 Bulletin 2019/04**

(73) Titulaires:
- **Ecole Centrale De Lille**
  **59651 Villeneuve d'Ascq (FR)**
- **Ecole Centrale De Lille**
  **59650 Villeneuve d'Ascq (FR)**
- **Centre National de la Recherche Scientifique**
  **75016 Paris (FR)**
- **Alma Mater Studiorum - Università di Bologna**
  **40126 Bologna (IT)**

(72) Inventeurs:
- **SANTARELLI, Francesco**
  **60019 Senigallia (IT)**
- **WOJCIESZAK, Robert**
  **59800 Lille (FR)**
- **PAUL, Sébastien**
  **59158 Thun Saint Amand (FR)**
- **DUMEIGNIL, Franck**
  **59491 Villeneuve D'Ascq (FR)**
- **CAVANI, Fabrizio**
  **41121 Modena (IT)**

(74) Mandataire: **Lavoix**
**2, place d'Estienne d'Orves**
**75441 Paris Cedex 09 (FR)**

(56) Documents cités:
**WO-A1-2013/096998**

- **NAVNEET KUMAR GUPTA ET AL: "Hydrotalcite-supported gold-nanoparticle-catalyzed highly efficient base-free aqueous oxidation of 5-hydroxymethylfurfural into 2,5-furandicarboxylic acid under atmospheric oxygen pressure", GREEN CHEMISTRY, vol. 13, no. 4, 2011, page 824, XP055071260, ISSN: 1463-9262, DOI: 10.1039/c0gc00911c**
- **ZEHUI ZHANG ET AL: "Recent Advances in the Catalytic Synthesis of 2,5-Furandicarboxylic Acid and Its Derivatives", ACS CATALYSIS, vol. 5, no. 11, 6 novembre 2015 (2015-11-06), pages 6529-6544, XP055278280, US ISSN: 2155-5435, DOI: 10.1021/acscatal.5b01491**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne un procédé catalytique hétérogène pour la préparation d'acide furoïque ou de l'un de ses dérivés à partir du furfural ou de l'un de ses dérivés en phase liquide.

L'acide furoïque (également nommé acide 2-furoïque, ou acide furane-2-carboxylique, ou encore acide alpha-furoïque) est un composé important pour l'industrie et peut être employé dans différents domaines d'application.

Notamment, dans le domaine de l'alimentaire, l'acide furoïque peut être utilisé comme conservateur dans les étapes de pasteurisation et de stérilisation, agissant ainsi comme un agent bactéricide et fongicide. Il peut également être utilisé en tant qu'agent aromatisant.

Dans le domaine de la recherche biomédicale, ce composé peut être utile dans la préparation de nylons.

L'acide furoïque permet aussi d'obtenir des esters d'acide furoïque et est fréquemment utilisé en tant qu'intermédiaire dans les industries chimique, pharmaceutique et agrochimique.

Enfin, il pourrait, à terme, tenir un rôle important dans le domaine des technologies optiques car les cristaux organiques polaires qu'il forme à l'état solide pourraient être des éléments clés des futures technologies photoniques permettant le stockage d'images et d'informations (Uma et al., Optik - International Journal for Light and Electron Optics, 2013, 124 (17), 2754-2757).

Parmi les dérivés d'acide furoïque, on peut citer notamment l'acide 2,5-furane dicarboxylique (également connu sous l'abréviation FDCA) qui peut être particulièrement utile dans les réactions de polymérisation afin d'obtenir, par exemple, des polyesters, des polyamides et des polyuréthanes. Il peut être aussi utilisé en pharmacologie.

L'acide furoïque peut aussi être facilement hydrogéné vers l'acide tétrahydro-furoïque, un intermédiaire très important dans l'industrie pharmaceutique.

Il existe donc un réel intérêt pour un procédé de préparation de dérivés d'acide furoïque qui soit économiquement avantageux.

Une approche conventionnelle consiste à oxyder le furfural en milieu alcalin en présence d'un catalyseur. Il est à noter que la mise en œuvre d'un milieu alcalin est nécessaire pour atteindre de bonnes performances catalytiques dans la réaction d'oxydation.

A titre illustratif de cette approche peuvent notamment être citées les oxydations en milieu alcalin du furfural à l'aide de catalyseurs métalliques en présence d'oxygène gazeux ($O_2$) (Tian et al., Molecules, 2008, 13(4), 948-957; Harrisson et al., Org. Synth., 1956, 36, 36; et le document JP26001111B4 de Terai et al.) ou à l'aide de sels de chromate (Hurd et al., J. Am. Chem. Soc., 1933, 55(3), 1082-1084 ; et Chakraborty et al., Synthetic Communications, 1980, 10(12), 951-956), ou bien en présence de peroxyde d'hydrogène ($H_2O_2$) (Corma et al., Chem. Rev., 2007, 107(6), 2411-2502) ou encore à l'aide de catalyseurs bimétalliques Pt-Pb supportés sur carbone (Corma et al., Chem. Rev., 2007, 107(6), 2411-2502 ; Verdeguer et al., J. Chem. Techn. Biotechnol., 1994, 61, 97-102 ; et Verdeguer et al., J. Chem. Techn. Biotechnol., 1994, 112, 1-11).

Bien que ces procédés conduisent, pour la plupart d'entre eux, à des rendements élevés en sels d'acide furoïque, ils ne sont malheureusement pas totalement satisfaisants.

Ainsi, ils ont pour inconvénient majeur de requérir, consécutivement à l'étape d'oxydation, au moins une étape de séparation et de conversion pour obtenir l'acide furoïque libre à partir de son sel alcalin. Cette approche conduit donc à la production de grandes quantités de sels généralement peu ou pas valorisables.

Il a été montré également que les catalyseurs métalliques employés sont facilement empoisonnés durant cette étape, ce qui les rend inactifs. De plus, la lixiviation de particules métalliques responsable d'une désactivation progressive des catalyseurs est également très souvent constatée.

Il a donc été noté que la nécessité de réaliser la réaction en milieu alcalin a pour effet secondaire indésirable la désactivation du catalyseur notamment en raison de la dégradation du support du catalyseur.

Ainsi, dans la pratique industrielle, l'acide furoïque est synthétisé en utilisant des catalyseurs $AgO/Cu_2O$. Cependant, un tel procédé souffre de la nécessité d'une forte charge en catalyseur, de l'utilisation d'un milieu dilué, et de l'existence de réactions secondaires. Le furfural subit non seulement l'oxydation en acide furoïque, mais aussi des réactions secondaires résultant du clivage du cycle furanique. En outre, le catalyseur doit être périodiquement régénéré dans la mesure où la phase $Cu_2O$ n'est pas stable.

Une autre approche de synthèse chimique de l'acide furoïque impose une réaction de Canizzaro préliminaire à partir du furfural dans une solution aqueuse de NaOH permettant d'obtenir l'alcool furfurylique et le 2-furanecarboxylate de sodium. L'étape suivante est la réaction du 2-furanecarboxylate de sodium avec de l'acide sulfurique pour obtenir l'acide furoïque. L'inconvénient majeur de ce procédé est la limitation du rendement théorique en acide furoïque à 50% et la génération en grande quantité d'hydrogénosulfate de sodium qui doit être éliminé du mélange réactionnel. WO2013096998 décrit un procédé de production d'un mélange d'acide 2,4-furanedicarboxylique et d'acide 2,5-furanedicarboxylique en milien alcalin.

L'acide furoïque peut être également synthétisé via des procédés biotechnologiques (Perez et al., African Journal of Biotechnology, 2009, 8(10), 2279-2282 ; Eilers et al., Planta, 1970, 94, 253-264 ; Luna et al., Rev. Mex.

Ciencias Farmacéuticas, 1997, 28, 17-19 ; et les documents CU22371A1 et WO9308293A1) ayant recours à l'action de certains microorganismes ou champignons parmi lesquels on peut citer les champignons tels que ceux de l'espèce *Neurospora crassa* et *Neurospora ascospora,* les levures telles que les *Saccharomyces cerevisiae,* et les bactéries telles que celles du genre *Acetobacter, Bacillus, Zooglea, Nocardia* et *Pseudomonas.*

Ainsi, à titre d'exemple, l'acide furoïque peut être préparé par oxydation du furfural en utilisant une préparation microbienne biocatalytique avec *Nocardia corallina* B-276 (Perez et al., African Journal of Biotechnology, 2009, 8(10), 2279-2282). Les expériences impliquant cette conversion microbienne ont abouti à des rendements élevés, à savoir 88 % à partir du furfural. L'oxydation avec *Nocardia corallina* a été considérée comme intéressante dans la mesure où l'utilisation de la plupart des autres microorganismes conduit à l'obtention de deux produits d'oxydation, à savoir l'acide et l'alcool correspondants. En outre, aucune destruction du cycle furanique n'est constatée. Toutefois, comme explicité ci-après, des inconvénients demeurent.

Les procédés actuellement considérés pour préparer de l'acide furoïque à partir du furfural ne donnent donc pas totalement satisfaction. Dans le cas des procédés biotechnologiques, les principaux inconvénients sont la complexité de mise en œuvre du procédé, la séparation des produits finaux du mélange des réactifs et également la nécessité d'utiliser des substrats de haute pureté ainsi qu'un milieu très dilué. En outre, dans le cas des cellules de *Nocardia corallina* B-276 précitées, le rendement en acide furoïque n'est que de 88 % seulement et est obtenu après 8 heures de réaction.

Les inconvénients les plus importants des procédés chimiques existants sont notamment liés à la nécessité de travailler en milieu alcalin. Comme évoqué précédemment, cette contrainte a pour effet indésirable d'une part, de nécessiter un traitement consécutif des produits secondaires et/ou intermédiaires et d'autre part, d'affecter la stabilité de certains des catalyseurs.

Il existe donc un intérêt tout particulier pour un procédé en catalyse hétérogène dénué des inconvénients précités.

Ainsi, l'un des objectifs de l'invention est de proposer un procédé en catalyse hétérogène, permettant de produire directement l'acide furoïque ou l'un de ses dérivés libre dans l'eau (et non sous la forme de sel alcalin) avec un rendement très élevé.

Un autre objectif de l'invention est de fournir un procédé en catalyse hétérogène ne requérant pas de travailler en milieu alcalin et donc permettant de s'affranchir du phénomène de dégradation du support du catalyseur et la perte de résidus métalliques généralement rencontrés dans des conditions alcalines.

Un autre objectif de la présente invention est de mettre à disposition un procédé en catalyse hétérogène permettant d'obtenir des rendements élevés en acide furoïque et avantageusement dans un temps de réaction réduit.

Un autre objectif de la présente invention est de proposer un procédé mettant en œuvre un catalyseur hétérogène pouvant être facilement recyclé sans requérir de traitement préalable.

Ainsi, la présente invention concerne un procédé de préparation d'acide furoïque ou de l'un de ses dérivés de formule (I) :

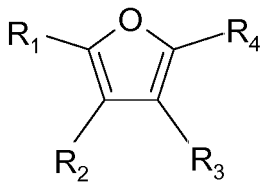

(I)

dans laquelle $R_1$, $R_2$, $R_3$ et $R_4$ représentent, indépendamment les uns des autres, un atome d'hydrogène, un groupe $C_1$-$C_6$ alkyle linéaire ou ramifié, un groupe -C(=O)-H ou un groupe -COOH,
à condition qu'au moins l'un des groupes $R_1$, $R_2$, $R_3$ et $R_4$ soit un groupe -COOH,
par oxydation catalytique hétérogène du furfural ou de l'un de ses dérivés de formule (II) :

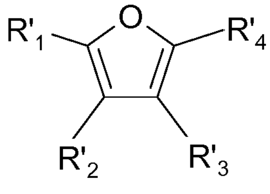

(II)

dans laquelle $R'_1$, $R'_2$, $R'_3$ et $R'_4$ représentent, indépendamment les uns des autres, un atome d'hydrogène, un

groupe $C_1$-$C_6$ alkyle linéaire ou ramifié, ou un groupe -C(=O)-H,
à condition qu'au moins l'un des groupes $R'_1$, $R'_2$, $R'_3$ et $R'_4$ soit un groupe -C(=O)-H,
caractérisé en ce que ladite oxydation est réalisée en présence d'un catalyseur supporté à base de nanoparticules d'or et dans un milieu aqueux non alcalin.

On entend par « oxydation » au sens de la présente invention, la conversion d'au moins une fonction aldéhyde -C(=O)-H comprise dans la formule (II) en au moins une fonction carboxylique -COOH comprise dans la formule (I).

Dans le cadre de la présente invention, un milieu aqueux non alcalin (ou milieu aqueux non basique) désigne un milieu de pH non alcalin c'est-à-dire de pH inférieur à 8 et de préférence au plus égal à 6.

Selon un mode de réalisation préféré, ce milieu aqueux est dénué de solvant organique.

Selon un mode de réalisation particulièrement préféré ce milieu aqueux consiste en de l'eau à titre de milieu solvant.

Dans le cadre de la présente invention, un groupe $C_1$-$C_6$ alkyle désigne un groupe alkyle comprenant de 1 à 6 atomes de carbone. Un tel groupe alkyle peut être linéaire ou ramifié et peut être choisi parmi méthyle, éthyle, propyle, isopropyle, butyle, isobutyle, tertio-butyle, pentyle, hexyle.

Selon un mode de réalisation particulier, le procédé selon l'invention permet d'obtenir un dérivé d'acide furoïque de formule (I) dans laquelle au plus deux des groupes $R_1$, $R_2$, $R_3$ et $R_4$ représentent un groupe -COOH.

Ainsi, selon ce mode de réalisation, le procédé selon l'invention peut conduire à l'obtention d'un dérivé d'acide furoïque de formule (I) dans laquelle $R_1$ et $R_2$, ou $R_1$ et $R_3$, ou $R_1$ et $R_4$, ou $R_2$ et $R_3$ représentent chacun un groupe -COOH et les deux autres groupes restants représentent, indépendamment les uns des autres, un atome d'hydrogène ou un groupe $C_1$-$C_6$ alkyle linéaire ou ramifié, de préférence un atome d'hydrogène.

Selon un mode de réalisation préféré, le procédé selon l'invention permet d'obtenir un dérivé d'acide furoïque de formule (I) dans laquelle $R_1$ et $R_4$ représentent chacun un groupe -COOH, et $R_2$ et $R_3$ représentent un atome d'hydrogène. Il s'agit alors de l'acide 2,5-furane dicarboxylique.

Selon un autre mode de réalisation, le procédé selon l'invention permet d'obtenir un dérivé d'acide furoïque de formule (I) dans laquelle $R_1$ représente un groupe -C(=O)-H ou un groupe -COOH ; $R_2$ et $R_3$ représentent, indépendamment l'un de l'autre, un atome d'hydrogène ou un groupe $C_1$-$C_6$-alkyle linéaire ou ramifié ; et $R_4$ est un groupe -COOH.

Selon un autre mode de réalisation particulier, le procédé selon l'invention permet d'obtenir un dérivé d'acide furoïque de formule (I) dans laquelle seulement l'un des groupes $R_1$, $R_2$, $R_3$ et $R_4$ est un groupe -COOH, et les trois autres groupes restants sont, indépendamment les uns des autres, un atome d'hydrogène ou un groupe $C_1$-$C_6$ alkyle linéaire ou ramifié.

Selon un autre mode de réalisation préféré, le procédé selon l'invention permet d'obtenir un dérivé d'acide furoïque de formule (I) dans laquelle $R_4$ est un groupe -COOH, et $R_1$, $R_2$, et $R_3$ représentent un atome d'hydrogène. Il s'agit alors de l'acide furoïque luimême.

Certes, des procédés d'oxydation du furfural utilisant des catalyseurs à base de nanoparticules d'or supportées sur du dioxyde de zirconium ($ZrO_2$) ou du dioxyde de cerium ($CeO_2$) en présence de méthanol sont bien connus mais ces réactions bien que réalisées en l'absence de base (milieu non alcalin), conduisent au furoate de méthyle et non à l'acide furoïque directement (Signoretto et al., Catalysts, 2013, 3, 656-670 ; Manzoli et al., Journal of Catalysis, 2015, Volume 330, 465-473 ; et Pinna et al., Catalysis Today, 2013, Volume 203, 196-201).

Comme il ressort de la partie expérimentale qui suit, le procédé selon l'invention est particulièrement avantageux à plusieurs titres.

Plus précisément, il permet d'accéder à un rendement très élevé en acide furoïque ou en l'un de ses dérivés à savoir un rendement de l'ordre de 98 %.

La conversion d'un dérivé de furfural de formule (II) en son dérivé d'oxydation de formule (I), en l'occurrence l'acide furoïque pour le furfural, est réalisée en une unique étape en présence d'oxygène gazeux en présence d'un catalyseur supporté à base d'or.

Le procédé selon l'invention met à profit des formulations catalytiques très efficaces, et réactives en milieu non basique.

Cet avantage permet d'accéder directement à la forme acide du composé de formule (II) et non à son sel alcalin. L'étape consécutive de conversion de ce sel en acide n'est donc plus requise et la production d'une grande quantité de sels peu ou pas valorisables est évitée.

En outre, les inventeurs ont découvert qu'aucun phénomène de lixiviation ne se produit pendant les tests catalytiques avec ce type de catalyseur.

En effet, les teneurs en or dans les solutions après 4 heures et 15,5 heures de réaction ont été mesurées par ICP-OES (mesures par spectrométrie à émission optique ICP, analyse à plasma induit). Elles se sont avérées en dessous de la limite de détection de l'appareil d'analyse indiquant que le passage d'or du catalyseur solide vers la solution (phénomène connu sous le nom de lixiviation) n'a pas lieu pour les catalyseurs convenant à la présente invention.

Selon une variante de réalisation, le procédé de l'invention comprend au moins les étapes consistant à :

(a) disposer d'une solution aqueuse non alcaline contenant au moins un dérivé du furfural de formule (II) ;
(b) mettre en contact ledit dérivé de formule (II) du milieu (a) avec de l'oxygène gazeux en présence d'au moins une quantité catalytiquement efficace de nanoparticules d'or supportées et dans des conditions non alcalines propices à l'oxydation dudit dérivé de furfural de formule (II) en dérivé d'acide furoïque de formule (I).

De manière préférée, l'étape (b) est réalisée sous agitation et sous une pression de l'ordre de 15 bars (soit $15.10^5$ Pa) en chauffant l'ensemble à une température comprise entre 70 °C et 150 °C, de préférence entre 90 °C à 120 °C, encore plus préférentiellement de 110 °C.
Le temps (ou durée) de réaction est ajusté pour obtenir un rendement en dérivé d'acide furoïque de formule (I) au moins égal à 80 %.
Ce temps de réaction peut avantageusement n'être que de 1 à 4 heures.
Selon un mode de réalisation particulier, le catalyseur utilisé est de l'or supporté sur du dioxyde de zirconium.
Selon un autre mode de réalisation particulier, le catalyseur utilisé est de l'or supporté sur hydrotalcite.
De manière avantageuse, comme illustré dans la partie expérimentale ci-après, le procédé selon l'invention permet d'obtenir un dérivé d'acide furoïque de formule (I) et plus particulièrement de l'acide furoïque avec une conversion, un rendement, une sélectivité et un bilan carbone élevés.
Au sens de la présente invention, les termes « taux de conversion » ou « conversion » désignent le rapport du nombre de moles de dérivé de furfural de formule (II) ayant réagi divisé par le nombre de moles de dérivé de furfural de formule (II) initialement introduit.
Par « sélectivité », on entend le rapport du nombre de moles de dérivé d'acide furoïque de formule (I) obtenu en fin de réaction divisé par le nombre de moles de dérivé de furfural de formule (II) ayant réagi.
Par « rendement », on entend le rapport du nombre de moles de dérivé d'acide furoïque de formule (I) obtenu en fin de réaction divisé par le nombre de moles de dérivé de furfural de formule (II) initialement introduit.
Par « bilan carbone », on entend le rapport du nombre d'atomes de carbone présents dans le réacteur en fin de réaction divisé par le nombre d'atomes de carbone présents initialement dans le réacteur.
Un autre avantage de la présente invention est que les conditions opératoires sont simples à mettre en œuvre et permettent l'obtention directe du dérivé d'acide furoïque de formule (I) libre de tout sel, du fait de l'absence de base dans le milieu réactionnel.
L'absence de base dans le milieu réactionnel permet également d'éviter la dégradation du support de catalyseur et la lixiviation de particules métalliques dans la phase liquide, ce qui rend le catalyseur recyclable pour plusieurs usages successifs en réacteur fermé ou stable sous flux en réacteur ouvert.
La présente invention vise également une composition non alcaline comprenant au moins du furfural et des nanoparticules d'or supportées.
Selon une variante de réalisation cette composition contient en outre un dérivé de furfural de formule (II) distinct du furfural et, le cas échéant, de l'eau.
Avantageusement, la composition est non alcaline.

## PROCEDE SELON L'INVENTION

### Définition des nanoparticules d'or

Les nanoparticules d'or convenant à l'invention sont bien connues et sont déjà couramment utilisées en chimie en tant que catalyseurs pour des réactions de type hydrogénation ou oxydation ainsi que notamment en optique, en électronique, en pharmacologie, en diagnostic ou en thérapie.
Pour la plupart de ces applications, les nanoparticules sont fixées à un support solide.
On connaît différents procédés de préparation de nanoparticules d'or, notamment en milieu confiné minéral ou en milieu confiné organique.
La préparation de nanoparticules d'or en milieu confiné minéral peut être effectuée dans des suspensions inorganiques (titane, silice, argile), par réduction d'un précurseur d'or en présence d'un catalyseur tel que décrit notamment par K. Nakamura et al. (J. Chem. Eng. Jap., 2001, 34, 1538-1544). La préparation de nanoparticules d'or au sein d'une matrice de silice portant des groupes hydroxyles, par réduction spontanée d'un précurseur d'or est décrite par P. Mukherjee et al. (Chem. Mater., 2002, 14, 1678-1684), et par T. Yokohama et al. (Journal of Colloid and Interface Science, 2001, 233, 112-116).
Les nanoparticules d'or convenant à l'invention ont une taille comprise entre 3 nm et 15 nm, de préférence entre 5 nm et 10 nm.
Comme énoncé précédemment, les particules d'or sont supportées.
A titre illustratif des supports convenant à l'invention, peuvent notamment être cités le dioxyde de zirconium ($ZrO_2$) et l'hydrotalcite.

Ainsi, selon un mode de réalisation, un catalyseur convenant à l'invention est de l'or supporté sur dioxyde de zirconium ou sur hydrotalcite.

Selon un mode de réalisation particulier, le catalyseur utilisé est de l'or supporté sur du dioxyde de zirconium.

Le pourcentage en masse d'or dans le catalyseur $Au/ZrO_2$ pour l'oxydation du furfural est compris entre 1 % et 7 % en poids, et est de préférence égal à 3 % en poids, et ce dans le cas du dioxyde de zirconium, utilisé comme support, de faible surface spécifique à savoir inférieure ou égale à 10 $m^2/g$.

Comme démontré dans la partie expérimentale ci-après, ce pourcentage de 3 % en poids permet d'obtenir une sélectivité optimale.

Un pourcentage en masse d'or relativement faible permet de disperser l'or à la surface du $ZrO_2$ et d'augmenter la quantité de sites actifs. Pour des pourcentages plus élevés, la formation d'agrégats d'or à la surface est possible, ce qui peut entrainer la formation d'un catalyseur moins actif.

Lorsque le catalyseur utilisé est $Au/ZrO_2$, le ratio molaire furfural/Au pour l'oxydation du furfural est compris entre 6 et 34, et de préférence ce ratio molaire est de 6.

Selon un autre mode de réalisation particulier, le catalyseur utilisé est de l'or supporté sur hydrotalcite.

Le pourcentage en masse d'or dans le catalyseur Au/hydrotalcite pour l'oxydation du furfural est compris entre 1 % et 3 % en poids, et est de préférence égal à 2 % en poids, et ce dans le cas de l'hydrotalcite, utilisé comme support, de faible surface spécifique à savoir inférieure ou égale à 10 $m^2/g$.

Comme exposé dans les exemples ci-après, ce pourcentage de 2 % en poids permet d'obtenir non seulement un rendement mais également une sélectivité en acide furoïque élevés.

Lorsque le catalyseur utilisé est Au/hydrotalcite, le ratio molaire furfural/Au pour l'oxydation du furfural est compris entre 22 et 50, de préférence ce ratio molaire est de 22.

Le catalyseur supporté selon l'invention peut être préparé selon tout procédé conventionnel.

Par exemple, un catalyseur supporté conforme à l'invention peut être élaboré en dissolvant une quantité requise (24,8 mg) d'hydrate d'acide chloroaurique (également nommé hydrate d'acide tétrachloroaurique) notamment commercialisé sous la dénomination $HAuO_4$ par la société Alpha Aesar dans une quantité appropriée d'eau (20 mL). D'autre sels d'or peuvent être également utilisés pour ce type de préparation comme les nitrates, notamment $Au(NO_3)_3$ ou les chlorures tels que notamment $AuCl_3$ ou $AU_2Cl_6$.

Puis à cette solution, une quantité appropriée (1 g) de support tel que du dioxyde de zirconium ou de l'hydrotalcite est ajoutée, et le mélange est agité à une température comprise entre 25 °C et 50 °C, de préférence à température ambiante (25 °C) pendant une durée d'au moins 10 minutes.

Ensuite, une quantité (2 mL) d'hydrazine ($N_2H_4$), par exemple celle commercialisée en solution aqueuse à 78-82 % par la société Sigma Aldrich (hydrate d'hydrazine) est injectée dans la solution. La réduction de l'or se produit alors spontanément.

La solution est par la suite soumise à une agitation pendant une durée comprise entre 30 et 60 minutes, de préférence pendant 40 minutes à une température comprise entre 25 °C et 50 °C, de préférence à température ambiante (25 °C) jusqu'à l'obtention d'un précipité qui est filtré puis lavé de manière appropriée.

Le catalyseur est obtenu sous forme solide puis séché dans un four à une température comprise entre 60 °C et 100 °C, de préférence à 80 °C pendant une durée comprise entre 6 et 14 heures, de préférence pendant une nuit.

Un exemple de préparation d'un catalyseur convenant à l'invention est plus particulièrement décrit dans l'exemple 1 ci-après.

## Conditions réactionnelles

L'oxydation peut être mise en œuvre de toute manière connue de l'homme du métier, notamment sous pression d'oxygène ou d'air, de préférence sous pression d'oxygène.

De préférence, la pression d'oxygène ou d'air mise en œuvre est telle que le rapport molaire $O_2$/dérivé de furfural de formule (II) est supérieur à 2.

La pression peut notamment être de 10 à 20 bars (soit $10.10^5$ à $20.10^5$ Pa) d'air, de préférence de 15 bars (soit $15.10^5$ Pa) d'air.

La pression partielle en oxygène peut être de 5 à 20 bars (soit $5.10^5$ à $20.10^5$ Pa), de préférence de 10 à 15 bars (soit $10.10^5$ à $15.10^5$ Pa), plus particulièrement de 15 bars (soit $15.10^5$ Pa).

Typiquement, la température de la réaction pour l'oxydation du dérivé de furfural de formule (II), en particulier du furfural, est comprise entre 70 °C et 150 °C, en particulier entre 90 °C et 120 °C, et de préférence est de 110 °C.

Toutefois, une température au-delà de 120°C est non privilégiée dans la mesure où elle est susceptible de provoquer la dégradation du furfural ou/et du produit, la formation de différents composés carbonés ainsi qu'une diminution du bilan carbone de la réaction. Or, le dépôt de carbone sur les catalyseurs métalliques peut entrainer la désactivation du catalyseur.

La durée de la réaction pour l'oxydation du dérivé de furfural de formule (II), en particulier du furfural, est

comprise entre 1 heure et 15,5 heures, de préférence entre 2 heures et 4 heures. Comme détaillé dans la partie expérimentale ci-après, il a été observé qu'un temps de réaction plus long que 4 heures n'augmente plus significativement le rendement de cette réaction et qu'après une durée de 15,5 heures, le furfural devient instable et la quantité en produits secondaires n'est plus négligeable.

Le procédé peut être mis en œuvre en mode continu ou en batch.

Dans un mode de réalisation avantageux, le procédé selon l'invention est mis en œuvre en batch.

D'une manière générale, le procédé selon l'invention peut être appliqué industriellement à l'oxydation du furfural et de ses dérivés pour obtenir l'acide carboxylique correspondant libre de tout sel.

Les expressions « *compris entre* ... *et* ... » et « *allant de* ... *à* ... » doivent se comprendre bornes incluses, sauf si le contraire est spécifié.

Les exemples qui suivent permettront de mieux comprendre l'invention, sans toutefois présenter un caractère limitatif.

## EXEMPLES

### EXEMPLE 1 : Préparation de catalyseurs convenant à l'invention

a) Catalyseur 3% Au/$ZrO_2$

66,5 mg d'hydrate d'acide chloroaurique (Au 49 % min, de la société Alpha Aesar, 99,9 %) sont dissous dans 20 mL d'eau. A cette solution, 997 mg de dioxyde de zirconium, monoclinique avec une structure de baddeleyite de la société Sigma Aldrich, sont ajoutés. Le mélange est ensuite agité à température ambiante (25 °C) pendant 10 minutes.

2 mL d'hydrazine ($N_2H_4$, solution aqueuse à 80 %, de la société Sigma Aldrich) sont ensuite injectés dans la solution. La réduction de l'or se produit alors spontanément.

La solution est agitée pendant 40 minutes à température ambiante (25 °C) jusqu'à l'obtention d'un précipité pourpre. Le précipité ainsi obtenu est ensuite filtré sous vide et lavé 3 fois avec de l'eau (3 fois 20 mL) et 1 fois avec de l'acétone (20 mL).

Le solide ainsi obtenu est par la suite séché dans un four à 80 °C pendant une nuit.

b) Catalyseur 2% Au/hydrotalcite

44,4 mg d'hydrate d'acide chloroaurique (Au 49 % min, de la société Alpha Aesar, 99,9 %) sont dissous dans 20 mL d'eau. A cette solution, 912 mg d'hydrotalcite, synthetisé au laboratoire avec une faible surface spécifique de 10 $m^2/g$ sont ajoutés. Le mélange est ensuite agité à température ambiante (25 °C) pendant 10 minutes.

2 mL d'hydrazine ($N_2H_4$, solution aqueuse à 80 %, de la société Sigma Aldrich) sont ensuite ajoutés à la solution. La réduction de l'or se produit alors spontanément.

La solution est agitée pendant 40 minutes à température ambiante (25 °C) jusqu'à l'obtention d'un précipité pourpre. Le précipité ainsi obtenu est ensuite filtré sous vide et lavé 3 fois avec de l'eau (3 fois 20 mL) et 1 fois avec de l'acétone (20 mL).

Le solide ainsi obtenu est par la suite séché dans un four à 80 °C pendant une nuit.

### EXEMPLE 2 : Procédure générale pour l'oxydation d'un dérivé de furfural de formule (II) et plus particulièrement du furfural.

La procédure détaillée ci-dessous est la procédure générale utilisée pour les tests catalytiques dont les résultats sont exposés dans l'exemple 3 de la présente invention.

Les tests catalytiques sont effectués dans un réacteur autoclave de 50 mL équipé d'un thermocouple (Top Industrie Autoclave 2456). La procédure pour un test standard est détaillée ci-après.

La quantité est ajustée en fonction du test à effectuer, par exemple 50 mg, (de 10 à 150 mg) de furfural sont ajoutés à de l'eau distillée (10 mL) et laissés sous agitation magnétique pendant 10 minutes. 9 mL de la solution de furfural sont ensuite ajoutés au réacteur autoclave sous pression atmosphérique et à température ambiante (25°C).

100 mg de catalyseur (nature variable selon le test réalisé) sont ensuite ajoutés au mélange réactionnel à température ambiante (25 °C). Les catalyseurs convenant à l'invention qui sont testés, à savoir Au/$ZrO_2$ et Au/hydrotalcite sont ceux préparés selon le protocole de l'exemple 1.

10 mL d'eau distillée sont à nouveau ajoutés dans le réacteur à température ambiante (25 °C). Le réacteur est ensuite fermé et l'agitation magnétique est fixée à 900 tours par minute à température ambiante (25 °C). Puis, le réacteur est purgé à trois reprises avec de l'oxygène. La pression en $O_2$ est ensuite fixée à $15.10^5$ Pa (soit 15 bars) et le réacteur est fermé. La température est ensuite fixée à la valeur souhaitée en fonction du test à réaliser, à savoir 90 °C, 110 °C

et 120 °C, comme indiqué dans les exemples ci-après.

Le temps de réaction imposé pour ces tests peut être également variable selon le paramètre testé, comme précisé ci-après, il peut être de 1 heure, 2 heures, 4 heures, voire 15,5 heures.

Puis, le réacteur est refroidi à température ambiante (25 °C) et la solution résultante est prélevée du réacteur, centrifugée pour la séparer des particules de catalyseur solide résiduel et analysée par HPLC (chromatographie en phase liquide à haute performance) dont le protocole est détaillé ci-après.

Analyse HPLC

Le mélange réactionnel est filtré en utilisant un filtre HPLC (2,5 microns), puis est dilué 5 fois avec de l'eau. L'analyse par chromatographie liquide est effectuée sur une chaîne HPLC Shimadzu UFLC-MS 20-20 équipée d'une colonne Phenomenex Synergi 2,5 μm Hydro-RP 100 Å. La colonne est purgée à 0,5 mL/min à température ambiante (25 °C) avec une solution aqueuse d'acide trifluoroacétique à 0,1 % comme phase mobile, pendant 12 minutes. Les temps de rétention pour chaque composé sont vérifiés en utilisant des standards commerciaux. La conversion, les rendements et la sélectivité sont déterminés par la méthode de la courbe d'étalonnage (réalisée pour chaque composé).

Les conversions sont calculées comme suit :

$$\frac{Furfural_{Tinitial} - Furfural_{Tfinal}}{Furfural_{Tinitial}} \times 100$$

Alors que le rendement est calculé comme suit :

$$\frac{Produit_{Tfinal}}{Furfural_{Tinitial}} \times 100$$

La vitesse de réaction est calculée comme suit :

$$\frac{Furfural_{Tinitial} - Furfural_{Tfinal}\ (\mathrm{mmol})}{temps\ (\mathrm{min}) \cdot masse\ Au\ \ (\mathrm{mg})}$$

EXEMPLE 3 : Performances catalytiques observées avec **un** catalyseur supporté à base d'or

3.1 Influence de la température

Le procédé d'oxydation tel que décrit ci-dessus est mis en œuvre avec le catalyseur Au/$ZrO_2$ conforme à l'invention tel que synthétisé en exemple 1a). Le pourcentage en masse d'or dans ce catalyseur est de 3% en poids.

La pression en $O_2$, la vitesse d'agitation et la quantité de catalyseur imposées sont celles indiquées dans l'exemple 2. La quantité de furfural utilisée est de 50 mg, et le temps de réaction est de 1 heure.

Les températures réactionnelles testées sont 90 °C, 110 °C et 120 °C.

Pour chacune de ces températures de réaction, le taux de conversion du furfural, le rendement en acide furoïque, la sélectivité en acide furoïque et le bilan carbone sont récapitulés dans le tableau 1 ci-dessous.

**Tableau 1**

| Température (°C) | Taux de conversion du furfural (%) | Rendement en acide furoïque (%) | Sélectivité en acide furoïque (%) | Bilan Carbone (%) |
|---|---|---|---|---|
| 90 | 10,7 | 6,1 | 57,5 | 95,5 |
| 110 | 35,0 | 29,7 | 84,7 | 95,0 |
| 120 | 46,0 | 36,5 | 79,2 | 90,4 |

Il ressort que la meilleure sélectivité est observée avec une température de réaction de 110 °C.

3.2 Influence de la concentration en Au

Un test préliminaire, hors invention, est réalisé en présence de $ZrO_2$ seul, c'est-à-dire sans nanoparticules d'or. Il convient de noter que la réaction d'oxydation du furfural n'a pas lieu.

Puis, le procédé d'oxydation tel que décrit ci-dessus est mis en œuvre avec le catalyseur $Au/ZrO_2$ conforme à l'invention. Quatre pourcentages différents en masse d'or ont été testés pour ce catalyseur, à savoir 1 %, 3 %, 5 % et 7 % en poids. Les catalyseurs sont préparés selon le protocole de synthèse décrit en exemple 1a), éventuellement adapté selon le pourcentage pondéral souhaité.

La pression en $O_2$, la vitesse d'agitation et la quantité de catalyseur imposées sont celles indiquées dans l'exemple 2. La quantité de furfural utilisée est de 50 mg, la température réactionnelle est de 110 °C, et le temps de réaction est de 4 heures.

Pour chacun des pourcentages en masse d'or, le ratio molaire furfural/Au, le taux de conversion du furfural, le rendement en acide furoïque, la sélectivité en acide furoïque et le bilan carbone sont récapitulés dans le tableau 2 ci-dessous.

**Tableau 2**

| Catalyseur | Ratio molaire furfural/Au | Taux de conversion du furfural (%) | Rendement en acide furoïque (%) | Sélectivité en acide furoïque (%) | Bilan Carbone (%) |
|---|---|---|---|---|---|
| 1 % $Au/ZrO_2$ | 100 | 7,0 | 3,9 | 55,5 | 96,9 |
| 3% $Au/ZrO_2$ | 30 | 35,0 | 29,7 | 84,7 | 95,0 |
| 5 % $Au/ZrO_2$ | 20 | 45,1 | 35,4 | 78,5 | 90,3 |
| 7% $Au/ZrO_2$ | 14 | 38,8 | 32,1 | 82,7 | 93,3 |

Il ressort que lorsque la charge en or augmente, la conversion en furfural augmente également, mais, en même temps, l'impact sur la sélectivité est plutôt limité, au moins pour des charges supérieures à 3 % en poids.

La meilleure sélectivité est observée avec $Au/ZrO_2$ 3 % en poids.

3.3 Influence du temps de réaction

Le procédé d'oxydation tel que décrit ci-dessus est mis en œuvre avec le catalyseur $Au/ZrO_2$ conforme à l'invention tel que synthétisé en exemple 1a). Le pourcentage en masse d'or dans ce catalyseur est de 3 % en poids.

La pression en $O_2$, la vitesse d'agitation et la quantité de catalyseur imposées sont celles indiquées dans l'exemple 2. La quantité de furfural utilisée est de 50 mg, et la température réactionnelle est de 110 °C.

Les temps réactionnels testés sont 1 heure, 2 heures, 4 heures et 15,5 heures.

Pour chacune de ces durées, le taux de conversion du furfural, le rendement (nommé Rdt) et la sélectivité (nommée Sélect.) en acide furoïque, en produits secondaires tels que la 2(5H)-furanone, l'acide maléique, et le dioxyde de carbone ainsi que et le bilan carbone sont récapitulés dans le tableau 3 ci-dessous.

**Tableau 3**

| Durée (h) | 1 | | 2 | | 4 | | 15,5 | |
|---|---|---|---|---|---|---|---|---|
| | Conversion (%) | | Conversion (%) | | Conversion (%) | | Conversion (%) | |
| Taux de conversion du furfural (%) | 35,0 | | 51,7 | | 68,0 | | 91,9 | |
| | Rdt (%) | Sélect. (%) | Rdt (%) | Sélect. (%) | Rdt (%) | Sélect. (%) | Rdt (%) | Sélect. (%) |
| Acide furoïque | 29,7 | 84,7 | 45,0 | 87,0 | 54,1 | 79,6 | 50,0 | 54,5 |
| 2(5H)furanone | 0,2 | 0,4 | 0,9 | 1,7 | 1,4 | 2,1 | 8,5 | 9,2 |
| Acide maléique | 0,1 | 0,5 | 0,4 | 0,7 | 0,4 | 0,6 | 2,5 | 2,7 |

(suite)

| Durée (h) | 1 | | 2 | | 4 | | 15,5 | |
|---|---|---|---|---|---|---|---|---|
| | Conversion (%) | | Conversion (%) | | Conversion (%) | | Conversion (%) | |
| **Dioxyde de carbone** | 0,0 | 0,0 | 0,2 | 0,3 | 0,2 | 0,3 | 1,4 | 1,5 |
| **Bilan carbone (%)** | 95,0 | | 94,6 | | 87,9 | | 69,1 | |

Des temps de réaction trop long ont révélé l'instabilité du furfural. Après 2 heures, le bilan carbone et la sélectivité en acide furoïque diminuent. Après une durée de 15,5 heures, ces diminutions sont encore plus importantes et simultanément la quantité de produits secondaires augmente.

3.1 Influence de la nature du support

Le procédé d'oxydation tel que décrit ci-dessus est mis en œuvre avec différents catalyseurs à base de nanoparticules d'or, à savoir Au/$CeO_2$, Au/MgO, Au/hydrotalcite tel que synthétisé en exemple 1b) et Au/$ZrO_2$ tel que synthétisé en exemple 1a). Les catalyseurs Au/Ce02 et Au/MgO, selon l'invention, peuvent être préparés par toute méthode connue de l'homme du métier et notamment selon celles décrites en exemple 1 précité. Le pourcentage en masse d'or dans ces catalyseurs est de 2 % ou 3 % en poids, comme spécifié dans le tableau 4 ci-après.

La pression en $O_2$, la vitesse d'agitation et la quantité de catalyseur imposées sont celles indiquées dans l'exemple 2. La température réactionnelle est de 110 °C et le temps de réaction de 2 heures.

Quant à la quantité de furfural utilisée, elle est de 50 mg.

Pour chacun de ces catalyseurs, le taux de conversion du furfural, le rendement et la sélectivité en acide furoïque, et le bilan carbone sont récapitulés dans le tableau 4 ci-dessous.

**Tableau 4**

| Support | Taux de conversion du furfural (%) | Rendement en acide furoïque (%) | Sélectivité en acide furoïque (%) | Bilan Carbone (%) |
|---|---|---|---|---|
| **3 % Au/$CeO_2$ selon l'invention** | 56,6 | 38,4 | 67,8 | 84,3 |
| **3 % Au/MgO Selon l'invention** | 33,1 | 8,8 | 26,5 | 75,7 |
| **2 % Au/hydrotalcite selon l'invention** | 78,5 | 71,8 | 91,4 | 93,3 |
| **3 % Au/$ZrO_2$ selon l'invention** | 51,7 | 45,0 | 87,0 | 94,6 |

Il ressort que de faibles activités sont observées dans les cas de l'Au supporté sur Ce02 ou MgO.

Une comparaison entre les tests catalytiques réalisés avec différents supports montre que, en utilisant un catalyseur supporté de type hydrotalcite, de bien meilleures performances sont obtenues (conversion et sélectivité plus élevées).

3.2 Influence du ratio molaire furfural/Au

3.5.1 Catalyseur Au/$ZrO_2$ conforme à l'invention

Le procédé d'oxydation tel que décrit ci-dessus est mis en œuvre avec le catalyseur Au/$ZrO_2$ conforme à l'invention tel que synthétisé en exemple 1a). Le pourcentage en masse d'or dans ce catalyseur est de 3 % en poids.

La pression en $O_2$, la vitesse d'agitation et la quantité de catalyseur imposées sont celles indiquées dans l'exemple 2. La température réactionnelle est de 110 °C et le temps de réaction de 4 heures.

Quant à la quantité de furfural utilisée, elle est ajustée afin d'obtenir trois ratios molaires furfural/Au respectivement de 34, 18 et 6.

Pour chacun de ces ratios, le taux de conversion du furfural, le rendement et la sélectivité en acide furoïque, et le bilan carbone sont récapitulés dans le tableau 5 ci-dessous.

**Tableau 5**

| Ratio molaire furfural/Au | Taux de conversion du furfural (%) | Rendement en acide furoïque (%) | Sélectivité en acide furoïque (%) | Bilan Carbone (%) |
|---|---|---|---|---|
| 34 | 68,0 | 54,1 | 79,6 | 87,3 |
| 18 | 78,4 | 66,1 | 84,2 | 89,5 |
| 6 | 87,8 | 76,6 | 87,3 | 90,0 |

De ces résultats, il ressort que le rapport molaire Furfural/Au a une forte influence sur les performances.

En effet, plus ce ratio molaire est faible, plus la conversion, la sélectivité et par conséquent le rendement en acide furoïque sont élevés.

3.5.2 Catalyseur Au/hydrotalcite conforme à l'invention

Le procédé d'oxydation tel que décrit ci-dessus est mis en œuvre avec le catalyseur Au/hydrotalcite conforme à l'invention tel que synthétisé en exemple 1b). Le pourcentage en masse d'or dans ce catalyseur est de 2% en poids.

La pression en $O_2$, la vitesse d'agitation et la quantité de catalyseur imposées sont celles indiquées dans l'exemple 2. La température réactionnelle est de 110 °C et le temps de réaction de 2 heures.

Quant à la quantité de furfural utilisée, elle est ajustée afin d'obtenir un ratio molaire furfural/Au de 22.

Le taux de conversion du furfural, le rendement et la sélectivité en acide furoïque, et le bilan carbone sont récapitulés dans le tableau 6 ci-dessous.

**Tableau 6**

| Ratio molaire furfural/Au | 22 | |
|---|---|---|
| | **Conversion (%)** | |
| **Furfural** | 98,2 | |
| | **Rendement (%)** | **Sélectivité (%)** |
| **Acide furoïque** | 96,7 | 98,5 |
| **Bilan carbone** | 98,5 | |

Ainsi, l'utilisation d'un catalyseur Au/hydrotalcite 2% en poids permet d'obtenir un rendement élevé en acide furoïque avec une sélectivité élevée.

**EXEMPLE 4 : Analyses à plasma induit des pertes de résidus métalliques éventuelles**

Les analyses à plasma induit (ICP) de la solution de réaction pour les essais (tests catalytiques effectués avec le catalyseur 3 % Au/$ZrO_2$ conforme à l'invention tel que synthétisé en exemple la) après 1 et 15,5 heures ont confirmé qu'aucune perte de résidus métalliques n'a eu lieu au cours de la réaction.

Les mesures par spectrométrie à émission optique ICP (ICP-OES) sont effectuées sur un spectromètre Agilent 720-ES. Les échantillons sont préparés par digestion en utilisant de l'eau régale. La quantité d'or dans la solution est déterminée en utilisant les courbes de calibration obtenues avec les solutions standards commerciales.

Le tableau 7 ci-dessous récapitule les données relevées, notamment l'intensité moyenne et l'intensité RSD (en anglais : Relative Standard Déviation, coefficient de variation) après 1 heure (blanc) et 15,5 heures de réaction (test catalytique).

**Tableau 7**

| | **Au 211,068** |
|---|---|
| **Blanc = référence (après 1 heure de réaction)** | |
| Intensité moyenne | 14,286 |
| Intensité % RSD | 26,781 |
| **Mesuré après 15,5 heures de réaction** | |
| Intensité moyenne | 12,123 |
| Intensité % RSD | 50,195 |
| Ratio Intensité/Intensité du blanc | 0,85 |

**Revendications**

**1.** Procédé de préparation d'acide furoïque ou de l'un de ses dérivés de formule (|) :

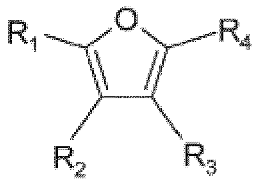
(I)

dans laquelle $R_1$, $R_2$, $R_3$ et $R_4$ représentent, indépendamment les uns des autres, un atome d'hydrogène, un groupe C1-C6 alkyle linéaire ou ramifié, un groupe -C(=O)-H ou un groupe -COOH,
à condition qu'au moins l'un des groupes $R_1$, $R_2$, $R_3$ et $R_4$ soit un groupe -COOH,
par oxydation catalytique hétérogène du furfural ou de l'un de ses dérivés de formule (II) :

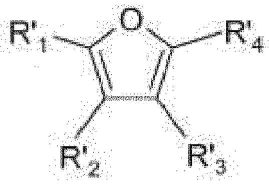
(II)

dans laquelle $R'_1$, $R'_2$, $R'_3$ et $R'_4$ représentent, indépendamment les uns des autres, un atome d'hydrogène, un groupe C1-C6 alkyle linéaire ou ramifié, ou un groupe -C(=O)-H,
à condition qu'au moins l'un des groupes $R'_1$, $R'_2$, $R'_3$ et $R'_4$ soit un groupe -C(=O)-H,
**caractérisé en ce que** ladite oxydation est réalisée en présence d'un catalyseur supporté à base de nanoparticules d'or et dans un milieu aqueux non alcalin de pH inférieur à 8.

**2.** Procédé selon la revendication 1, dans lequel $R_1$ et $R_4$ représentent chacun un groupe - COOH, et $R_2$ et $R_3$ représentent un atome d'hydrogène.

**3.** Procédé selon la revendication 1, dans lequel $R_4$ est un groupe -COOH, et $R_1$, $R_2$ et $R_3$ représentent un atome d'hydrogène.

**4.** Procédé selon l'une quelconque des revendications précédentes, **caractérisé en ce que** le milieu aqueux non alcalin de pH inférieur à 8a un pH au plus égal à 6.

**5.** Procédé selon l'une quelconque des revendications précédentes, **caractérisé en ce que** le milieu aqueux est dénué

de solvant organique.

**6.** Procédé selon l'une quelconque des revendications précédentes, **caractérisé en ce que** le milieu aqueux consiste en de l'eau à titre de milieu solvant.

**7.** Procédé selon l'une quelconque des revendications précédentes, **caractérisé en ce qu'**il comprend au moins les étapes consistant à :

(a) disposer d'une solution aqueuse non alcaline contenant au moins un dérivé de furfural de formule (II) ;
(b) mettre en contact ledit dérivé de formule (II) du milieu (a) avec de l'oxygène gazeux en présence d'au moins une quantité catalytiquement efficace de nanoparticules d'or supportées et dans des conditions non alcalines propices à l'oxydation dudit dérivé de furfural de formule (II) en dérivé d'acide furoïque de formule (I).

**8.** Procédé selon l'une quelconque des revendications précédentes, **caractérisé en ce que** l'oxydation est réalisée sous une pression partielle en oxygène comprise entre $5.10^5$ Pa et $20.10^5$ Pa, de préference entre $10.10^5$ Pa et $15.10^5$ Pa, plus particulièrement égale à $15.10^5$ Pa.

**9.** Procédé selon l'une quelconque des revendications précédentes, caractérisé en ce l'oxydation est réalisée à une température comprise entre 70 °C et 150 °C, de préférence entre 90 °C à 120 °C, encore plus préférentiellement de 110 °C.

**10.** Procédé selon l'une quelconque des revendications précédentes, **caractérisé en ce que** le catalyseur est de l'or supporté sur dioxyde de zirconium ou sur hydrotalcite.

**11.** Procédé selon l'une quelconque des revendications précédentes, **caractérisé en ce que** le catalyseur est de l'or supporté sur hydrotalcite.

**12.** Procédé selon l'une quelconque des revendications 1 à 10, **caractérisé en ce que** le catalyseur est de l'or supporté sur dioxyde de zirconium.

**13.** Procédé selon la revendication 10 ou 12, **caractérisé en ce que** le pourcentage en masse d'or dans le catalyseur $Au/ZrO_2$ pour l'oxydation du furfural est compris entre 1 % et 7 % en poids, et de préférence de 3 % en poids.

**14.** Procédé selon la revendication 13, **caractérisé en ce que** le dioxyde de zirconium, utilisé comme support, possède une surface spécifique inférieure ou égale à 10 m2/g.

**15.** Procédé selon l'une quelconque des revendications 12 à 14, **caractérisé en ce que** le ratio molaire furfural/Au pour l'oxydation du furfural est compris entre 6 et 34, et de préférence le ratio furfural/Au est de 6.

**16.** Procédé selon la revendication 10 ou 11, **caractérisé en ce que** le pourcentage en masse d'or dans le catalyseur Au/hydrotalcite pour l'oxydation du furfural est compris entre 1 % et 3 % en poids, et est de préférence égal à 2 % en poids.

**17.** Procédé selon la revendication 16, **caractérisé en ce que** l'hydrotalcite, utilisé comme support, possède une surface spécifique inférieure ou égale à 10 m2/g.

**18.** Procédé selon l'une quelconque des revendications 11, 16 et 17, **caractérisé en ce que** le ratio molaire furfural/Au pour l'oxydation du furfural est compris entre 22 et 50, et de préférence le ratio furfural/Au est de 22.

**19.** Procédé selon l'une quelconque des revendications précédentes, **caractérisé en ce que** la taille des nanoparticules d'or dans le catalyseur pour l'oxydation du furfural est comprise entre 3 nm et 15 nm, et est de préférence comprise entre 5 nm et 10 nm.

**20.** Procédé selon l'une quelconque des revendications précédentes, **caractérisé en ce qu'**il peut être mis en œuvre en mode continu ou en batch.

**21.** Composition non alcaline comprenant au moins du furfural et des nanoparticules d'or supportées.

**22.** Composition selon la revendication 21 comprenant en outre un dérivé de furfural de formule (II) distinct du furfural et éventuellement de l'eau.

## Patentansprüche

**1.** Verfahren zur Herstellung von Furonsäure oder von einem Derivat von Formel (I):

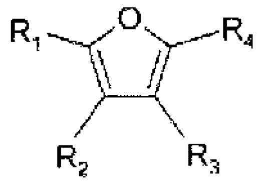

(I)

wobei $R_1$; $R_2$, $R_3$ und $R_4$ unabhängig voneinander ein Wasserstoffatom, eine lineare oder verzweigte C1-C6-Alkylgruppe, eine -C(=O)-H-Gruppe oder eine -COOH-Gruppe darstellen,
mit der Maßgabe, dass mindestens eine der Gruppen $R_1$, $R_2$, $R_3$ und $R_4$ eine -COOH-Gruppe ist,
durch heterogene katalytische Oxidation von Furfural oder von einem Derivat von Formel (II):

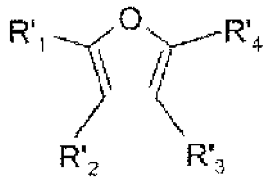

(II)

wobei $R'_1$; $R'_2$, $R'_3$ und $R'_4$ unabhängig voneinander ein Wasserstoffatom, eine lineare oder verzweigte C1-C6-Alkylgruppe oder eine -C(=O)-H-Gruppe darstellen,
mit der Maßgabe, dass mindestens eine der Gruppen $R'_1$, $R'_2$, $R'_3$ und $R'_4$ eine -C(=O)-H-Gruppe ist,
**dadurch gekennzeichnet, dass** die Oxidation in Anwesenheit eines Katalysators basierend auf Goldnanopartikeln auf einem Träger und in einem nicht alkalischen wässrigen Medium mit einem pH-Wert von weniger als 8 durchgeführt wird.

**2.** Verfahren nach Anspruch 1, wobei $R_1$ und $R_4$ jeweils eine -COOH-Gruppe darstellen und $R_2$ und $R_3$ ein Wasserstoffatom darstellen.

**3.** Verfahren nach Anspruch 1, wobei $R_4$ eine -COOH-Gruppe ist und $R_1$; $R_2$ und $R_3$ ein Wasserstoffatom darstellen.

**4.** Verfahren nach einem der vorherigen Ansprüche, **dadurch gekennzeichnet, dass** das nicht-alkalische wässrige Medium mit einem pH-Wert von weniger als 8 einen pH-Wert von höchstens 6 aufweist.

**5.** Verfahren nach einem der vorherigen Ansprüche, **dadurch gekennzeichnet, dass** das wässrige Medium frei von organischen Lösungsmitteln ist.

**6.** Verfahren nach einem der vorherigen Ansprüche, **dadurch gekennzeichnet, dass** das wässrige Medium aus Wasser als Lösungsmittelmedium besteht.

**7.** Verfahren nach einem der vorherigen Ansprüche, **dadurch gekennzeichnet, dass** es mindestens die Schritte umfasst, die aus Folgendem bestehen:

(a) Bereitstellen einer nicht-alkalischen wässrigen Lösung, die mindestens ein Furfuralderivat von Formel (II) enthält;
(b) Inkontaktbringen des Derivats von Formel (II) des Mediums (a) mit gasförmigem Sauerstoff in Anwesenheit von mindestens einer katalytisch wirksamen Menge an Goldnanopartikeln auf einem Träger und unter nicht-alkalischen Bedingungen, die die Oxidation des Furfuralderivats von Formel (II) zu dem Furosäurederivat von

Formel (I) begünstigen.

**8.** Verfahren nach einem der vorherigen Ansprüche, **dadurch gekennzeichnet, dass** die Oxidation unter einem Sauerstoffpartialdruck zwischen $5{,}10^5$ Pa und $20{,}10^5$ Pa, vorzugsweise zwischen $10{,}10^5$Pa und $15{,}10^5$Pa, insbesondere gleich $15{,}10^5$ Pa, durchgeführt wird.

**9.** Verfahren nach einem der vorherigen Ansprüche, **dadurch gekennzeichnet, dass** die Oxidation bei einer Temperatur zwischen 70 °C und 150 °C, vorzugsweise zwischen 90 °C und 120 °C, noch bevorzugter 110 °C, durchgeführt wird.

**10.** Verfahren nach einem der vorherigen Ansprüche, **dadurch gekennzeichnet, dass** der Katalysator Gold auf Zirkoniumdioxid oder auf Hydrotalcit getragen ist.

**11.** Verfahren nach einem der vorherigen Ansprüche, **dadurch gekennzeichnet, dass** der Katalysator Gold auf Hydrotalcit getragen ist.

**12.** Verfahren nach einem der Ansprüche 1 bis 10, **dadurch gekennzeichnet, dass** der Katalysator Gold auf Zirkoniumdioxid getragen ist.

**13.** Verfahren nach Anspruch 10 oder 12, **dadurch gekennzeichnet, dass** der Masseprozentsatz an Gold in dem $Au/ZrO_2$-Katalysator für die Oxidation von Furfural zwischen 1 und 7 Gewichtsprozent, vorzugsweise 3 Gewichtsprozent, liegt.

**14.** Verfahren nach Anspruch 13, **dadurch gekennzeichnet, dass** das als Träger verwendete Zirkoniumdioxid eine spezifische Oberfläche von 10 $m^2/g$ oder weniger besitzt.

**15.** Verfahren nach einem der Ansprüche 12 bis 14, **dadurch gekennzeichnet, dass** das Molverhältnis Furfural/Au für die Oxidation von Furfural zwischen 6 und 34 liegt und vorzugsweise das Verhältnis Furfural/Au 6 ist.

**16.** Verfahren nach Anspruch 10 oder 11, **dadurch gekennzeichnet, dass** der Masseprozentsatz an Gold in dem Au/Hydrotalcit-Katalysator für die Oxidation von Furfural zwischen 1 und 3 Gewichtsprozent liegt und vorzugsweise 2 Gewichtsprozent ist.

**17.** Verfahren nach Anspruch 16, **dadurch gekennzeichnet, dass** der als Träger verwendete Hydrotalcit eine spezifische Oberfläche von weniger als oder gleich wie 10 $m^2/g$ besitzt.

**18.** Verfahren nach einem der Ansprüche 11, 16 und 17, **dadurch gekennzeichnet, dass** das Molverhältnis Furfural/Au für die Oxidation von Furfural zwischen 22 und 50 liegt, und vorzugsweise das Verhältnis Furfural/Au 22 ist.

**19.** Verfahren nach einem der vorherigen Ansprüche, **dadurch gekennzeichnet, dass** die Größe der Goldnanopartikel in dem Katalysator für die Oxidation von Furfural zwischen 3 nm und 15 nm liegt und vorzugsweise zwischen 5 nm und 10 nm ist.

**20.** Verfahren nach einem der vorherigen Ansprüche, **dadurch gekennzeichnet, dass** es in einem kontinuierlichen oder chargenweisen Betrieb durchgeführt werden kann.

**21.** Nicht-alkalische Zusammensetzung, umfassend mindestens Furfural und Goldnanopartikel auf einem Träger.

**22.** Zusammensetzung nach Anspruch 21, ferner umfassend ferner ein Furfuralderivat von Formel (II), das sich von Furfural unterscheidet, und optional Wasser.

## Claims

**1.** Process for the preparation of furoic acid or of one of its derivatives of formula (I):

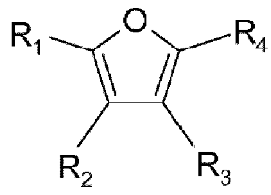

(I)

in which $R_1$, $R_2$, $R_3$ and $R_4$ represent, independently of each other, a hydrogen atom, a linear or branched $C_1$-$C_6$ alkyl group, a -C(=O)-H group or a -COOH group,
provided that at least one of $R_1$, $R_2$, $R_3$ and $R_4$ is a -COOH group,
by heterogeneous catalytic oxidation of furfural or a derivative thereof of formula (II):

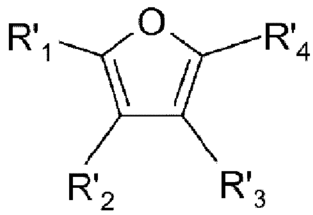

(II)

in which $R'_1$, $R'_2$, $R'_3$ and $R'_4$ represent, independently of each other, a hydrogen atom, a linear or branched $C_1$-$C_6$ alkyl group or a -C(=O)-H group,
provided that at least one of the $R'_1$, $R'_2$, $R'_3$ and $R'_4$ groups is a -C(=O)-H group,
**characterized in that** the oxidation is carried out in the presence of a supported catalyst based on gold nanoparticles and in a non-alkaline aqueous medium having a pH of less than 8.

**2.** Process according to claim 1, wherein $R_1$ and $R_4$ are each a -COOH group, and $R_2$ and $R_3$ are hydrogen.

**3.** Process according to claim 1, wherein $R_4$ is -COOH group, and $R_1$, $R_2$, and $R_3$ are hydrogen.

**4.** Process according to any one of the preceding claims, wherein the non-alkaline aqueous medium having a pH of less than 8 has a pH of at most 6.

**5.** Process according to any one of the preceding claims, wherein the aqueous medium is devoid of organic solvent.

**6.** Process according to any one of the preceding claims, wherein the aqueous medium consists of water as a solvent medium.

**7.** Process according to any one of the preceding claims, **characterized in that** it comprises at least the steps consisting of:

(a) providing a non-alkaline aqueous solution containing at least one furfural derivative of formula (II);
(b) contacting the derivative of formula (II) of the medium (a) with gaseous oxygen in the presence of at least a catalytically-effective amount of supported gold nanoparticles and under non-alkaline conditions conducive to the oxidation of the furfural derivative of formula (II) to the furoic acid derivative of formula (I).

**8.** Process according to any one of the preceding claims, wherein the oxidation is carried out under a partial pressure of oxygen of between $5.10^5$ Pa and $20.10^5$ Pa, preferably of between $10.10^5$ Pa and $15.10^5$ Pa, more preferably equal to $15.10^5$ Pa.

**9.** Process according to any one of the preceding claims, wherein the oxidation is carried out at a temperature between 70°C and 150°C, preferably between 90°C and 120°C, more preferably of 110°C.

**10.** Process according to any one of the preceding claims, wherein the catalyst is gold supported on zirconium dioxide or on hydrotalcite.

**11.** Process according to any one of the preceding claims, wherein the catalyst is gold supported on hydrotalcite.

**12.** Process according to any one of claims 1 to 10, wherein the catalyst is gold supported on zirconium dioxide.

**13.** Process according to claim 10 or 12, wherein the percentage by weight of gold in the catalyst $Au/ZrO_2$ for the oxidation of furfural is between 1% and 7% by weight, and preferably of 3% by weight.

**14.** Process according to claim 13, wherein zirconium dioxide, used as a support, has a specific surface of less than or equal to 10 $m^2/g$.

**15.** Process according to any one of claims 12 to 14, wherein the furfural/Au molar ratio for the oxidation of furfural is between 6 and 34, and preferably the furfural/Au ratio is 6.

**16.** Process according to claim 10 or 11, wherein the percentage by weight of gold in the Au/hydrotalcite catalyst for the oxidation of furfural is between 1% and 3% by weight, and preferably of 2% by weight.

**17.** Process according to claim 16, wherein the hydrotalcite, used as a support, has a specific surface less than or equal to 10 $m^2/g$.

**18.** Process according to any one of claims 11, 16 and 17, wherein the furfural/Au molar ratio for the oxidation of furfural is between 22 and 50, and preferably the furfural/Au ratio is 22.

**19.** Process according to any one of the preceding claims, wherein the size of the gold nanoparticles in the catalyst for the oxidation of furfural is between 3 nm and 15 nm, and is preferably between 5 nm and 10 nm.

**20.** Process according to any one of the preceding claims, wherein the method is implemented in continuous mode or in batch mode.

**21.** Non-alkaline composition comprising at least furfural and supported gold nanoparticles.

**22.** Composition according to claim 21, further comprising a furfural derivative of formula (II) different from furfural and optionally water.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- JP 26001111 B, Terai
- WO 2013096998 A
- CU 22371 A1
- WO 9308293 A1


### Littérature non-brevet citée dans la description


- **UMA et al.** *Optik - International Journal for Light and Electron Optics,* 2013, vol. 124 (17), 2754-2757
- **TIAN et al.** *Molecules,* 2008, vol. 13 (4), 948-957
- **HARRISSON et al.** *Org. Synth.,* 1956, vol. 36, 36
- **HURD et al.** *J. Am. Chem. Soc.,* 1933, vol. 55 (3), 1082-1084
- **CHAKRABORTY et al.** *Synthetic Communications,* 1980, vol. 10 (12), 951-956
- **CORMA et al.** *Chem. Rev.,* 2007, vol. 107 (6), 2411-2502
- **VERDEGUER et al.** *J. Chem. Techn. Biotechnol.,* 1994, vol. 61, 97-102
- **VERDEGUER et al.** *J. Chem. Techn. Biotechnol.,* 1994, vol. 112, 1-11
- **PEREZ et al.** *African Journal of Biotechnology,* 2009, vol. 8 (10), 2279-2282
- **EILERS et al.** *Planta,* 1970, vol. 94, 253-264
- **LUNA et al.** *Rev. Mex. Ciencias Farmacéuticas,* 1997, vol. 28, 17-19
- **SIGNORETTO et al.** *Catalysts,* 2013, vol. 3, 656-670
- **MANZOLI et al.** *Journal of Catalysis,* 2015, vol. 330, 465-473
- **PINNA et al.** *Catalysis Today,* 2013, vol. 203, 196-201
- **K. NAKAMURA et al.** *J. Chem. Eng. Jap.,* 2001, vol. 34, 1538-1544
- **P. MUKHERJEE et al.** *Chem. Mater.,* 2002, vol. 14, 1678-1684
- **T. YOKOHAMA et al.** *Journal of Colloid and Interface Science,* 2001, vol. 233, 112-116